# FASCICULE DE BREVET EUROPEEN

(11) **EP 1 212 336 B1**
(45) Date de publication et mention de la délivrance du brevet: **03.12.2003**
(21) Numéro de dépôt: 00958756.9
(22) Date de dépôt: 28.08.2000
(51) Int. Cl.: C07H 17/08, A61K 9/16, A61K 9/50

(54) **PROCEDE DE PREPARATION D'AGGLOMERATS SPHERIQUES DE TELITHROMYCINE ET LEUR APPLICATION DANS LA PREPARATION DE FORMES PHARMACEUTIQUES**
VERFAHREN ZUR HERSTELLUNG VON SPHÄRISCHE TELITHROMYCIN-AGGLOMERATE SOWIE DEREN VERWENDUNG ZUR VORBEREITUNG VON ARZNEIFORMEN
METHOD FOR THE PRODUCTION AND USE OF SPHERICAL TELITHROMYCIN CLUSTERS IN THE PREPARATION OF PHARMACEUTICAL FORMS

(30) Priorité: 26.08.1999 FR 9910810
(43) Date de publication de la demande: 12.06.2002
(73) Titulaire: Aventis Pharma S.A., 92160 Antony (FR)
(72) Inventeur: GODARD, Jean-Yves, F-93340 Le Raincy (FR); ROGNON, Valérie, F-93470 Coubron (FR)
(74) Mandataire: Vieillefosse, Jean-Claude
(86) Numéro de dépôt international: FR0002393
(87) Numéro de publication internationale: WO01014393

(56) Documents cités:
- EP-A- 0 130 160
- EP-A- 0 680 967
- GRAUL, A. ET AL: "HMR-3647, an antimicrobial ketolide" DRUGS FUTURE (1998), 23(6), 591-597, XP000909275
- PATENT ABSTRACTS OF JAPAN vol. 014, no. 539 (C-0782), 28 novembre 1990 (1990-11-28) & JP 02 227130 A (TAISHO PHARMACEUT CO LTD), 10 septembre 1990 (1990-09-10)
- GUILLAUME ET AL.: "Sperical crystallization of meprobamate" IL FARMACO, vol. 48, no. 4, 1993, pages 473-485, XP000906957 cité dans la demande

## Description

La présente invention a pour objet un procédé de préparation d'agglomérats sphériques de télithromycine et leur application dans la préparation de formes pharmaceutiques.

La télithromycine ou 11,12-dideoxy-3-de((2,6-dideoxy-3-C-méthyl-3-O-méthyl-alpha-L-ribohexopyranosyl)oxy)-6-O-méthyl-3-oxo-12,11-(oxycarbonyl((4-(4-(3-pyridinyl)-1H-imidazol-1-yl)butyl)imino))-érythromycine est un produit doué de propriétés antibiotiques de structure : 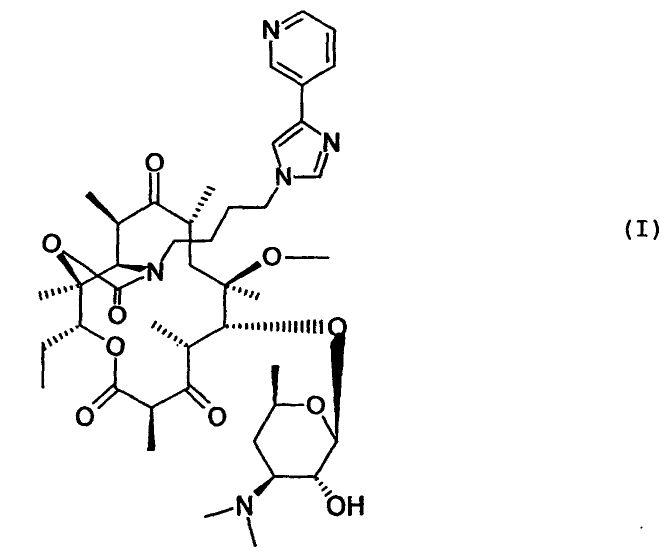 décrit et revendiqué dans le brevet européen 680967.

Par ailleurs, dans la référence A.Graul et al. Drugs of the future, 1998, 23(6) : 591-597 est décrite notamment une synthèse de la télithromycine, ainsi que des propriétés physiques et anti-bactériennes de ce composé, de même qu'une étude pharmacodynamique in vitro et in vivo.

La voie orale est une forme d'administration privilégiée pour ce produit. Certains patients, les enfants en particulier, ont des difficultés pour avaler les comprimés et les gélules et de ce fait, il est souhaitable de disposer d'autres formes d'administration, par exemple des suspensions orales, prêtes à l'emploi ou préparées extemporanément au moment de l'emploi.

La télithromycine est un principe actif qui présente un mauvais goût. Il faut donc préparer des formes galéniques qui masquent le goût du produit et conservent néanmoins une bonne biodisponibilité.

Les qualités physico-chimiques de la télithromycine sont telles qu'elles permettent la micro-encapsulation, c'est-à-dire l'enrobage du principe actif par un polymère ou un mélange de polymères.

La micro-encapsulation peut être réalisée par pulvérisation d'un polymère ou par polymérisation interfaciale ou par coacervation. Pour obtenir une bonne micro-encapsulation, il faut disposer de particules sphériques de principe actif, des particules qui ne soient ni trop petites, pour éviter qu'elles ne s'agglomèrent entre elles, ni trop grosses pour que la dissolution ne soit trop lente, les particules doivent être sphériques pour que le recouvrement du principe actif par le polymère soit correct et pour obtenir une bonne cinétique de libération du principe actif.

Les agglomérats sphériques sont obtenus par transformation directe des cristaux en amas de forme sphérique et l'invention a ainsi pour objet un procédé de préparation d'agglomérats sphériques caractérisé en ce que l'on prépare une suspension de cristaux de télithromycine, puis enrobe ces cristaux d'une phase insoluble en télithromycine qui cristallise progressivement.

L'invention a notamment pour objet un procédé de préparation caractérisé en ce que l'on utilise une solution de télithromycine dans l'acétone.

L'invention a plus particulièrement pour objet un procédé de préparation caractérisé en ce que la cristallisation a lieu dans un mélange acétone/éther isopropylique.

Dans un mode de réalisation préféré, la cristallisation est réalisée entre -5 et -15°C. La taille des agglomérats sphériques est contrôlée en ajustant la vitesse d'agitation.

A propos des agglomérats sphériques en général, on peut se reporter à l'article de Frederica Guillaume et Anne-Marie Guyot-Hermann Il Farmaco XLVIII 1993 pages 473 et suivantes.

Les agglomérats selon l'invention permettent une bonne micro-encapsulation et l'invention a notamment pour objet l'application caractérisée en ce que l'on entoure les agglomérats sphériques d'une couche de polymère pour obtenir la forme galénique recherchée, par exemple des micro-capsules.

Les agglomérats sphériques de télithromycine obtenus selon l'invention sont caractérisés en ce que la taille des particules est comprise entre 30 et 400 microns et notamment en ce que la taille médiane des particules est située entre 80 et 150 microns. Les agglomérats sphériques de télithromycine préférés sont caractérisés en ce que la taille médiane des particules est située vers 100 microns, c'est-à-dire caractérisé en ce que la moitié des agglomérats ont une taille inférieure à 100 microns.

L'exemple suivant illustre l'invention sans toutefois la limiter.

### EXEMPLE :

### a) Préparation de la solution acétonique

On introduit sous azote :
- télithromycine 64 g
- acétone pure anhydre 128 ml

On agite sous une légère surpression d'azote entre 19°C et 21°C et vérifie que la dissolution est totale.

On ajoute, si nécessaire, la quantité d'eau pour obtenir un produit à 2,9 %, on ajoute :
- eau déminéralisée 0,26 ml.

### b) Cristallisation

Dans un réacteur à double enveloppe muni d'un agitateur mécanique, d'une sonde thermométrique et d'une arrivée d'azote, on introduit sous azote :
- éther isopropylique 640 ml
- acétone pure anhydre 12,8 ml

On stabilise la température entre 19° et 21°C.

On introduit 5 % en masse de la solution acétonique tout en agitant à 350 tr/min.

Puis, tout en agitant à 350 tr/min, on amorce la cristallisation avec de la télithromycine micronisée 0,96 g mise en suspension par sonication dans :
éther isopropylique 3,2 ml

La cristallisation se développe immédiatement après amorçage.

On agite pendant 15 minutes à 20±1°C puis refroidit la suspension à -10±1°C en 30 minutes.

On introduit le reste de la solution acétonique :
solution acétonique de télithromycine 157,2 g

On agite encore pendant 1 h à -10°C.

### c) Isolement

On essore à fond et lave par clairçage à deux reprises avec à chaque fois :
éther isopropylique 64 ml.

On sèche en étuve à 40°C sous vide. On tamise sur une grille de 500 µm.

On obtient 50,4 g d'agglomérats sphériques de télithromycine.

### Granulométrie

La taille des particules est déterminée par diffraction laser à l'aide d'un granulomètre modèle HELOS SYMPATEC®.

Les résultats obtenus sont les suivants :
10 % des particules ont un diamètre < 77 microns
50 % des particules ont un diamètre < 107 microns
90 % des particules ont un diamètre < 166 microns.

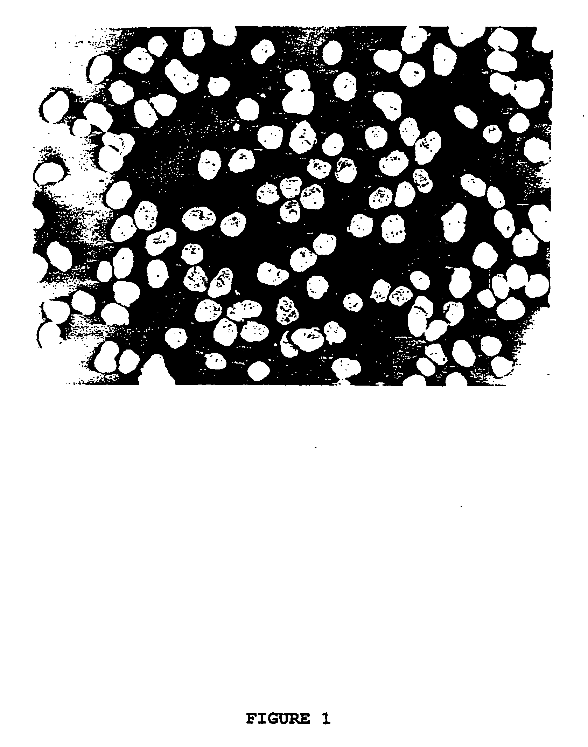

La figure 1 représente des agglomérats obtenus en opérant comme indiqué ci-dessus, l'échelle est
1 cm = 150 microns.

### Application

Le produit de l'exemple a été utilisé pour préparer par coacervation simple ou par pulvérisation directe d'un polymère approprié des micro-capsules destinées à la préparation de suspensions orales à préparer extemporanément.

Les suspensions préparées sont acceptées par les enfants et conservent une bonne cinétique de libération.

## Revendications

1. Procédé de préparation d'agglomérats sphériques de télithromycine, **caractérisé en ce que** l'on prépare une suspension de cristaux de télithromycine, puis enrobe ces cristaux d'une phase insoluble en télithromycine qui cristallise progressivement.

2. Procédé de préparation selon la revendication 1, **caractérisé en ce que** l'on utilise une solution de télithromycine dans l'acétone.

3. Procédé de préparation selon la revendication 1 ou 2, **caractérisé en ce que** la cristallisation a lieu dans un mélange acétone/éther isopropylique.

4. Procédé de préparation selon l'une quelconque des revendications 1 à 3, **caractérisé en ce que** la cristallisation est réalisée entre -5° et -15°C.

5. Application des agglomérats sphériques de télithromycine à la préparation d'une forme micro encapsulée, **caractérisée en ce que** l'on entoure les agglomérats sphériques d'une couche de polymère pour obtenir la forme galénique recherchée.

## Patentansprüche

1. Verfahren zur Herstellung von sphärischen Agglomeraten von Telithromycin, **dadurch gekennzeichnet, daß** man eine Suspension von Telithromycin-Kristallen herstellt, anschließend diese Kristalle mit einer in Telithromycin unlöslichen Phase umhüllt, die schrittweise kristallisiert.

2. Verfahren zur Herstellung nach Anspruch 1, **dadurch gekennzeichnet, daß** man eine Lösung von Telithromycin in Aceton verwendet.

3. Verfahren zur Herstellung nach Anspruch 1 oder 2, **dadurch gekennzeichnet, daß** die Kristallisation in einer Mischung von Aceton/Isopropylether stattfindet.

4. Verfahren zur Herstellung nach irgendeinem der Ansprüche 1 bis 3, **dadurch gekennzeichnet, daß** die Kristallisation bei einer Temperatur zwischen -5 °C und -15 °C realisiert wird.

5. Verwendung der sphärischen Agglomerate von Telithromycin zur Herstellung einer Mikrokapsel-Form, **dadurch gekennzeichnet, daß** man die sphärischen Agglomerate mit einer Polymerschicht umschließt, um die gesuchte galenische Form zu erhalten.

## Claims

1. Process for the preparation of spherical agglomerates of telithromycin, **characterized in that** a suspension of telithromycin crystals is prepared, then these crystals are coated with a phase which is insoluble in telithromycin, which gradually crystallizes.

2. Preparation process according to claim 1, **characterized in that** a solution of telithromycin in acetone is used.

3. Preparation process according to claim 1 or 2, **characterized in that** the crystallization takes place in an acetone/isopropyl ether mixture.

4. Preparation process according to any one of claims 1 to 3, **characterized in that** the crystallization is carried out between -5° and -15°C.

5. Use of the spherical agglomerates of telithromycin in the preparation of a micro-encapsulated form, **characterized in that** the spherical agglomerates are surrounded by a layer of polymer in order to obtain the sought galenic form.
